# FASCICULE DE BREVET EUROPEEN

(11) **EP 1 409 102 B1**
(45) Date de publication et mention de la délivrance du brevet: **05.04.2006**
(21) Numéro de dépôt: 00966205.7
(22) Date de dépôt: 27.09.2000
(51) Int. Cl.: B01D 11/02

(54) **PROCEDE ET INSTALLATION DE MISE A L'ETAT ADSORBE SUR UN SUPPORT POREUX DE COMPOSES ACTIFS CONTENUS DANS UN PRODUIT**
VERFAHREN UND VORRICHTUNG ZUR ADSORPTION VON AKTIVEN VERBINDUNGEN AUS EINEM PRODUKT AUF EINEM PORÖSEM TRÄGER
METHOD AND INSTALLATION FOR SETTING IN ADSORBED STATE ON A POROUS SUPPORT ACTIVE COMPOUNDS CONTAINED IN A PRODUCT

(30) Priorité: 27.09.1999 FR 9912005
(43) Date de publication de la demande: 21.04.2004
(73) Titulaire: Separex Société Anonyme, 54250 Champigneulles (FR)
(72) Inventeur: PERRUT, Michel, F-54000 Nancy (FR); MAJEWSKI, Wieslaw, F-54520 Laxou (FR)
(74) Mandataire: Puiroux, Guy
(86) Numéro de dépôt international: PCT/FR2000/002668
(87) Numéro de publication internationale: WO 2001/023064

(56) Documents cités:
- EP-A- 0 379 963
- WO-A-91/14373
- PATENT ABSTRACTS OF JAPAN vol. 14, no. 382, 17 août 1990 (1990-08-17) & JP 02 139003 A (NIPPON SANSO KK), 29 mai 1990 (1990-05-29)

## Description

La présente invention concerne un procédé et une installation permettant, industriellement, la mise à l'état adsorbé, sur un support poreux, de certains composés actifs contenus dans des produits naturels ou synthétiques. Plus précisément une telle opération sera effectuée après une phase préliminaire au cours de laquelle lesdits composés actifs seront extraits de ces produits à l'aide d'un solvant porté à pression supercritique, c'est-à-dire un fluide en état supercritique ou un liquide subcritique.

On sait en effet que les corps sont généralement connus sous trois états, à savoir solide, liquide ou gazeux et que l'on passe de l'un à l'autre en faisant varier la température et/ou la pression. Or il existe un point au-delà duquel on peut passer de l'état liquide à l'état gaz ou vapeur sans passer par une ébullition ou à l'inverse par une condensation, mais de façon continue : ce point est appelé le point critique.

On sait également qu'un fluide en état supercritique, c'est-à-dire un fluide qui est dans un état caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange, présente, pour de très nombreuses substances, un pouvoir solvant élevé sans commune mesure avec celui observé dans ce même fluide à l'état de gaz comprimé. Il en est de même des liquides dits "subcritiques" c'est-à-dire des liquides qui se trouvent dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange (voir à ce sujet l'article de Michel PERRUT - Les Techniques de l'Ingénieur "Extraction par fluide supercritique, J 2 770 - 1 à 12, 1999").

Les variations importantes et modulables du pouvoir solvant des fluides supercritiques sont d'ailleurs utilisées dans de nombreux procédés d'extraction (solide/fluide), de fractionnement (liquide/fluide), de chromatographie analytique ou préparative, de traitement des matériaux (céramiques, polymères,...). Des réactions chimiques ou biochimiques sont également réalisées dans de tels solvants. Il est à noter que les propriétés physicochimiques du dioxyde de carbone ainsi que ses paramètres critiques (pression critique : 7,4 MPa et température critique : 31°C) en font le solvant préféré dans de nombreuses applications, d'autant qu'il ne présente pas de toxicité et est disponible à très bas prix en très grande quantité. Solvant non polaire, le dioxyde de carbone porté à pression supercritique est parfois additionné d'un co-solvant constitué notamment par un solvant organique polaire qui a pour fonction de modifier le pouvoir solvant de façon notable, surtout vis-à-vis de molécules présentant une certaine polarité, l'éthanol étant souvent utilisé à cette fin. Toutefois, certains composés sont plus favorablement extraits par un hydrocarbure léger ayant de 2 à 8 atomes de carbone, et plus favorablement, de 2 à 4 atomes de carbone, à pression supercritique.

L'un des avantages principaux des procédés utilisant des fluides à pression supercritique en tant que solvants réside dans la facilité de réaliser la séparation entre le solvant et les extraits et solutés, ainsi qu'il a été décrit dans de nombreuses publications et, pour certains aspects importants de mise en oeuvre, dans le brevet français FR-A-2 584 618. Les propriétés intéressantes de ces fluides sont d'ailleurs utilisées depuis longtemps en extraction solide-fluide et fractionnement liquide-fluide, ainsi qu'il est décrit dans l'article cité précédemment.

Afin de séparer les substances restant dans le fluide avant son recyclage, il est connu d'utiliser un lit d'adsorbant qui va fixer ces substances et purifier le fluide, tel que décrit par exemple dans le brevet japonais JP-A-02139003.

On sait enfin que l'extraction de produits naturels par un fluide à pression supercritique conduit à des extraits de très grande qualité qui sont de plus en plus utilisés dans de nombreuses applications. Toutefois, ces extraits, comme d'ailleurs les extraits obtenus avec d'autres moyens comme par exemple l'extraction par solvant organique, se présentent souvent comme des produits très visqueux ou même pâteux, dont la manipulation n'est pas aisée ; si bien que leur incorporation dans des supports solides, le dosage et le mélange avec une matrice et, éventuellement d'autres principes actifs, au sein d'un excipient solide sont très difficiles. On est parfois contraint de les mettre en solution dans un solvant organique pour réaliser l'imprégnation d'un excipient solide, ce qui est regrettable puisque disparaît ainsi un avantage déterminant dans de nombreuses applications pour lesquelles il y a lieu d'éviter tout contact du produit avec un solvant organique.

La présente invention a pour but de proposer des moyens permettant, à des fins de production industrielle, d'extraire des principes actifs d'intérêt notamment pharmaceutique, cosmétologique, diététique, de matières premières diverses dans lesquelles ils se trouvent dilués, en concentration variable, selon l'origine de ces matières premières et la période de leur récolte, comme c'est toujours le cas pour les produits d'origine naturelle, et de fixer l'extrait obtenu au cours de l'extraction dans une matrice poreuse adéquate par imprégnation en une seule et même opération.

Suivant l'invention on couple l'opération d'extraction par fluide à pression supercritique, avec une seconde opération au cours de laquelle on réalise, de façon simultanée, la séparation de l'extrait mélangé au fluide solvant et l'imprégnation d'un milieu poreux par cet extrait.

La présente invention a ainsi pour objet un procédé de mise à l'état adsorbé, sur un support poreux, de composés contenus dans un produit, dans lequel, au cours d'une première étape, on réalise l'extraction des composés en mettant en contact avec le produit au moins un fluide solvant à pression supercritique conduisant à l'obtention d'un mélange d'extraits et de fluide solvant, caractérisé en ce que, dans une seconde étape on élimine l'eau contenue dans le mélange d'extraits et de fluide solvant, on règle les conditions de température et de pression de façon à obtenir, dans une enceinte, deux phases, à savoir une première phase essentiellement constituée du fluide solvant à l'état gazeux et une seconde phase constituée d'un mélange de liquides formé de fluide solvant et des extraits du produit, on fait percoler ces deux phases à travers un support poreux apte à adsorber les extraits, on vaporise le fluide solvant contenu dans la seconde phase.

On assurera l'élimination de l'eau en faisant percoler le mélange d'extraits et de fluide solvant sur un lit de produit adsorbant apte à fixer l'eau de façon sélective.

Le fluide solvant pourra être constitué de dioxyde de carbone pur, de protoxyde d'azote ou d'un hydrocarbure léger comptant de 2 à 4 atomes de carbone. Le fluide solvant pourra être pur ou éventuellement additionné d'un ou plusieurs co-solvants. Ainsi le fluide solvant pourra notamment être constitué d'un mélange de dioxyde de carbone avec au moins un co-solvant constitué d'un alcool et préférablement d'éthanol, d'une cétone et préférablement d'acétone, d'un ester et préférablement d'acétate d'éthyle.

Préférentiellement la première étape d'extraction pourra être réalisée à une pression comprise entre 7,4 MPa et 80 MPa, et préférablement entre 10 MPa et 40 MPa, et à une température comprise entre 0°C et 80°C. De même la percolation des deux phases à travers le support poreux pourra être effectuée à une pression comprise entre 1 MPa et 10 MPa, et préférablement entre 4 MPa et 8 Mpa, et à une température comprise entre 0°C et 80°C.

La présente invention a également pour objet une installation d'extraction/imprégnation du type comportant un extracteur contenant un produit dont on souhaite extraire les composés, qui est traversé à cet effet par au moins un fluide solvant à pression supercritique, caractérisé en ce qu'il comporte successivement, en aval de l'extracteur, des moyens d'élimination de l'eau contenue dans les composés extraits notamment constitués d'un lit d'absorbant sélectif, des moyens aptes à créer, dans une enceinte d'imprégnation contenant un milieu poreux, deux phases, à savoir une première phase essentiellement constituée du fluide solvant à l'état gazeux et une seconde phase constituée d'un mélange de liquides formé de fluide solvant et des extraits du produit, de façon à réaliser l'adsorption par le milieu poreux des composés extraits. L'enceinte d'imprégnation pourra comporter des moyens d'apport d'enthalpie, notamment constitués d'une double enveloppe à circulation de fluide caloporteur.

L'installation pourra également comporter, en aval de l'enceinte d'imprégnation, des moyens de condensation du fluide solvant.

Dans une variante de mise en oeuvre de l'invention l'extracteur pourra être constitué d'une colonne de fractionnement fonctionnant à contre-courant, adaptée au traitement de matières premières liquides.

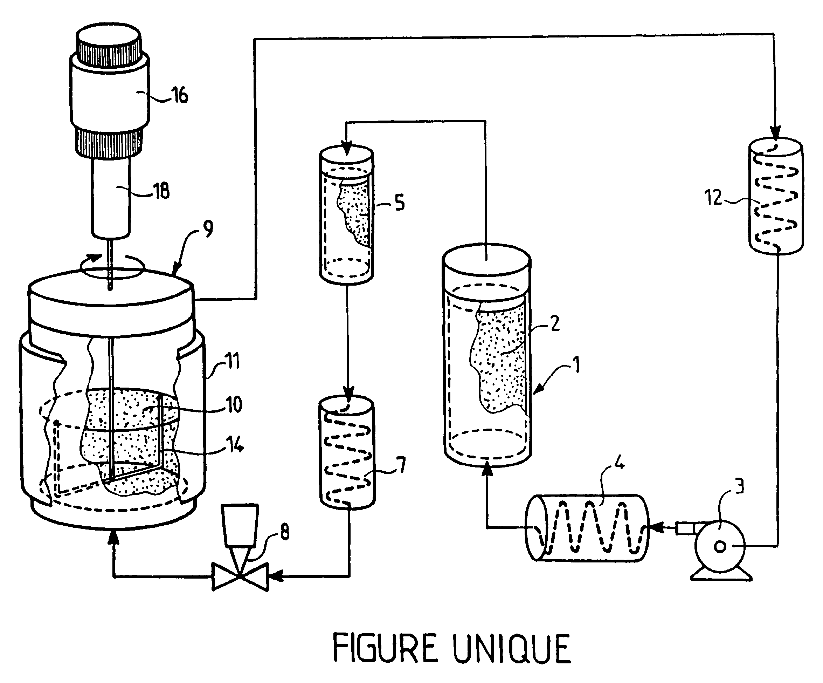

On décrira ci-après, à titre d'exemples non limitatifs, diverses formes d'exécution de la présente invention, en référence à la figure unique annexée qui représente de façon schématique une installation permettant de mettre en oeuvre le procédé suivant l'invention.

Comme il a été décrit en détail dans l'article et le brevet cités précédemment, on sait que les procédés d'extraction et de fractionnement utilisant un fluide à pression supercritique comprennent deux étapes successives: une première étape au cours de laquelle le fluide solvant est mis en contact avec la matière première à traiter, dans des conditions de pression et de température telles que son pouvoir solvant vis-à-vis des composés à extraire est élevé, et une seconde étape durant laquelle le fluide est placé dans des conditions de pression et de température telles que son pouvoir solvant est très faible vis-à-vis des produits qu'il a extraits de la matière première lors de l'étape antérieure, ce qui permet la séparation de ces extraits, le fluide étant ensuite recyclé. A la différence des mises en oeuvre classiques consistant à séparer les deux phases obtenues lors de cette seconde étape dans des séparateurs gravitaires ou inertiels, le procédé selon l'invention consiste à faire percoler le mélange de ces deux phases au sein d'un milieu poreux.

On a remarqué que si l'on opère sans précaution particulière, les extraits ne se répartissent pas de façon homogène sur et à l'intérieur du milieu poreux et, même en procédant à l'agitation de ce milieu poreux s'il est sous forme pulvérulente ou granulaire, par exemple à l'aide d'une turbine en rotation, on obtient en général des grumeaux de taille variable résultant de l'agglomération des particules du milieu poreux par les extraits qui, au lieu de pénétrer dans les pores, restent en surface sans répartition homogène. De plus, les produits naturels, susceptibles d'être avantageusement traités par un procédé de ce type, ayant toujours une certaine teneur en eau, les extraits contiennent toujours de l'eau dont la présence complique leur adsorption sur les supports poreux ou excipients car la plupart de ceux-ci ont une grande affinité pour l'eau, si bien que leur structure et leurs propriétés mécaniques sont gravement altérées par l'eau ainsi apportée par le fluide en même temps que les extraits visés.

Par contre, lorsque l'on piège l'eau, notamment par un adsorbant sélectif, et que l'on opère ensuite l'imprégnation du milieu poreux dans des conditions de pression et de température déterminées, on constate que, de façon surprenante, les extraits imprègnent ce milieu poreux de manière très homogène et reproductible, donnant par exemple naissance à une poudre non collante et présentant une bonne fluidité dans le cas où le milieu poreux initial présente également ces caractéristiques.

On décrira ci-après, en regard de la figure unique un exemple d'installation permettant de mettre en oeuvre le procédé suivant l'invention.

Cette installation est dérivée d'une unité classique d'extraction par fluide à pression supercritique destinée au traitement de matières solides en discontinu. Elle comprend un extracteur 1 contenant un panier 2 destiné à recevoir la matière première à traiter, et une pompe à membrane 3 qui distribue du dioxyde de carbone liquide à la pression de travail, au travers d'un échangeur de chaleur 4 permettant de chauffer le fluide à la température de travail. A la différence des installations classiques d'extraction par fluide à pression supercritique, le fluide sortant de l'extracteur 1 est conduit dans un récipient 5 qui contient un milieu poreux adsorbant sélectif de l'eau, tel que notamment du tamis moléculaire 3A. Le fluide qui est dans les mêmes conditions de pression et de température que celles régnant dans l'extracteur 1, percole à travers le milieu poreux où il abandonne l'eau qu'il contient.

Le récipient 5 est réuni à une vanne de détente 8 par l'intermédiaire d'un échangeur réchauffeur 7. La sortie de la vanne 8 est reliée au fond d'une enceinte d'imprégnation 9 qui contient le milieu poreux 10 dans lequel on souhaite adsorber les extraits et qui est pourvue de moyens de chauffage constitués par exemple d'une double enveloppe 11 dans laquelle circule un fluide caloporteur ce qui permet d'avoir une bonne vaporisation du fluide solvant. Dans ces conditions, on a donc en permanence, dans l'enceinte d'imprégnation 9, une phase gazeuse constituée par du fluide solvant et une phase liquide constituée des extraits et du fluide solvant.

Le milieu poreux 10 est choisi en fonction de l'utilisation ultérieure que l'on souhaite faire du produit final, que ce soit un milieu granulaire ou pulvérulent particulièrement adapté pour une utilisation en diététique, en pharmacie ou en cosmétique, ou un milieu massif. Dans le cas le plus fréquent où ce milieu se présente sous forme granulaire ou pulvérulente, une mise en oeuvre particulièrement avantageuse consiste à agiter ce milieu poreux au sein de l'enceinte d'imprégnation 9 par tout moyen adéquat, par exemple au moyen d'une turbine 14 mue par un moteur électrique 16 via un système d'entraînement magnétique 18.

La partie supérieure de l'enceinte 9 est réunie à la pompe 3 au travers d'un condenseur 12.

On peut bien entendu, suivant l'invention, utiliser tout autre système de séparation, et on pourrait remplacer l'extracteur 1 par une colonne de fractionnement permettant le traitement de matières premières à l'état liquide en continu ou en discontinu, le fluide sortant de la colonne étant traité de la même manière que celle décrite précédemment.

Dans une variante de l'invention, on utilise une pompe annexe pour introduire dans l'extracteur 1 un ou plusieurs co-solvants organiques permettant de modifier le pouvoir solvant et la polarité du fluide solvant. Souvent, on choisit d'additionner de l'éthanol qui peut être de qualité alimentaire ou CODEX selon la destination des produits ainsi élaborés. On peut aussi favorablement utiliser comme co-solvant un hydrocarbure léger ayant entre 2 et 8 atomes de carbone. Dans le cas où l'on utilise un co-solvant, on choisira naturellement un milieu poreux qui n'est pas altéré par ce co-solvant. De plus, en fin d'opération d'extraction-imprégnation, on prendra soin de balayer le milieu poreux avec du fluide solvant pur sans co-solvant afin d'éliminer le co-solvant adsorbé.

On a constaté que, dans ces conditions, l'on obtenait une excellente diffusion du fluide au travers des pores du milieu poreux et corrélativement l'entraînement dans ces derniers des extraits et leur adsorption dans les pores.

Un apport d'enthalpie est requis pour maintenir une quantité constante de fluide à l'état liquide au sein de l'enceinte d'imprégnation 9, à mesure qu'est injecté le fluide provenant de l'extraction et qu'est donc vaporisé un débit identique de fluide qui sort de cette enceinte à l'état gazeux. Cet apport d'enthalpie doit être soigneusement réglé afin d'éviter soit, s'il est insuffisant, l'accumulation de fluide liquéfié dans le récipient d'imprégnation qui finirait par ressortir sous cette forme liquide en emportant une partie des extraits, soit, s'il est trop important, la vaporisation totale du fluide et la précipitation non contrôlée des extraits.

Comme il sera illustré dans les exemples suivants de mise en oeuvre du procédé et de l'installation selon l'invention, il est surprenant de réaliser l'extraction et d'obtenir une imprégnation très homogène de différents excipients par les extraits en une seule opération, sans avoir jamais à manipuler les extraits eux-mêmes, ce qui ne peut qu'éviter tout risque de dégradation par oxydation à l'air ou par l'exposition à la chaleur, puisque tout contact avec l'air est évité et l'ensemble des opérations est conduit à une température voisine de l'ambiante.

On décrira ci-après plusieurs exemples de l'invention qui ont été mis en oeuvre avec l'installation précédemment décrite et dont les paramètres de fonctionnement étaient les suivants :

| | |
|---|---|
| Volume du panier 2 | : 0.5 l |
| Débit de la pompe 3 | : 1 à 5 kg/h |
| Pression de travail | : de l'ordre de 30 MPa |
| Température de travail | : entre 10°C et 80°C |
| Contenu du récipient 5 | : 100 g de zéolithe 3A |
| Volume de l'enceinte d'imprégnation 9 | : 4,5 l |
| Matériau poreux | : poudre de maltodextrine de qualité alimentaire obtenue par hydrolyse partielle d'amide de maïs. |
| Vitesse de rotation de la turbine 18 | 120 tours/minute |

### Exemple 1: Extraction et imprégnation de KAVA-KAVA:

Kava-kava est le nom local d'un arbuste sauvage des îles du Pacifique, identifié comme *Piper methysticum* ou *Piper wichmannu,* dont les racines contiennent des produits de grand intérêt pharmacologique appelés kavalactones, largement utilisés sous différentes formes comme tranquillisant et euphorisant naturel.

L'extraction a été conduite sur 100 g de poudre de racines séchées, broyées vers 200 µm environ, avec un débit de 3 kg/h de dioxyde de carbone à 25 MPa et 40°C, et a fourni un extrait qui se présente comme une pâte très visqueuse jaune foncé à odeur caractéristique. Après adsorption de l'eau le fluide a été détendu à 6 MPa et injecté par le bas dans l'enceinte d'imprégnation 9 contenant 100 g de maltodextrine. On a observé que le gaz comprimé sortant par le haut de l'enceinte 9 possédait une température constante voisine de 40°C en régime établi. Après avoir poursuivi l'opération pendant 510 min, l'enceinte d'imprégnation 9 a été décomprimée et l'on a récupéré dans celle-ci 114,8 g d'une poudre jaune vif, présentant l'odeur caractéristique de l'extrait de kava-kava et coulant sans problème en l'absence de tout grumeau ou agglomérat, idéale pour la fabrication de comprimés, en mélange éventuel avec un excipient ou d'autres principes actifs. Un échantillon de cette poudre a été réextrait au chloroforme et analysé par chromatographie en phase gazeuse. On a constaté que l'extrait fixé sur la maltodextrine était constitué de 89% en masse de kavalactones dont l'identification permet de vérifier que l'abondance relative de chacun de ces composés est conforme avec ce qui est trouvé dans un extrait classique. Ceci confirme donc à la fois la haute sélectivité de l'extraction par le dioxyde de carbone à pression supercritique et la fixation complète de l'extrait sur la maltodextrine.

### Exemple 2: Extraction et imprégnation de KAVA-KAVA:

On a procédé à une seconde opération dans des conditions identiques à celles utilisées dans l'exemple 1, à la différence que, cette fois, la masse initiale de maltodextrine était de 50 g seulement. Après 510 min d'opération on a obtenu 65,6 g de poudre dont les caractéristiques sont identiques à celles de la poudre obtenue à l'exemple 1, à la différence que sa couleur et son odeur sont plus intenses. L'analyse de l'extrait fixé a révélé un pourcentage en kavalactones de 91% en masse, avec une répartition entre les différents composés quasi identique à celle observée dans l'exemple précédent. Ceci montre que l'on peut charger la maltodextrine d'au moins 30% en masse d'extrait.

### Exemple 3: Extraction et imprégnation de KAVA-KAVA :

On a procédé à une troisième opération dans des conditions identiques à celles utilisées dans l'exemple 2, à la différence que, cette fois, on a choisi un milieu poreux composé d'un mélange intime de poudres de maltodextrine et de lécithine de soja de qualité alimentaire, à raison de 45 g de maltodextrine pour 5 g de lécithine. La masse initiale de milieu poreux a été fixée à 50 g. Après 510 min d'opération on a obtenu 64,2 g de poudre, dont les caractéristiques se sont révélées voisines de celles de la poudre obtenue à l'exemple 1, à la différence que sa couleur et son odeur sont plus intenses. L'analyse de l'extrait fixé a conduit à un pourcentage en kavalactones de 90% en masse, avec une répartition entre les différents composés quasi identique à celle observée dans l'exemple précédent. Cette poudre présente ainsi l'avantage, par rapport aux poudres obtenues dans les exemples 1 et 2, de se disperser plus facilement dans l'eau, donnant lieu à un trouble ressemblant à celui obtenu en diluant du pastis dans l'eau. On peut donc non seulement l'utiliser dans des formulations sèches, telles que les comprimés, mais également sous forme d'une poudre à délayer dans l'eau pour préparer une potion buvable.

### Exemple 4: Extraction et imprégnation d'un épice (Curcuma):

L'extraction a été conduite sur une masse totale de 800 g du Curcuma moulu, répartie en 4 lots de 200 g placés successivement dans l'extracteur 1 avec un débit de 2,4kg/h de dioxyde de carbone à 40°C et 29 MPa. Après élimination de l'eau ce dernier a été détendu à 5 MPa et injecté par le bas dans l'enceinte d'imprégnation 9 contenant une masse de 400 g de maltodextrine qui a été imprégnée successivement avec les extraits issus des quatre lots. Après avoir poursuivi l'opération pendant quatre périodes de 50 min chacune, l'on a récupéré 445 g d'une poudre orangée très homogène, présentant l'odeur et le goût caractéristique de l'extrait du Curcuma.

### Exemple 5: Extraction et imprégnation d'un mélange d'épices (Poivre noir + Paprika doux).

On a procédé à une opération d'extraction et imprégnation des épices dans des conditions identiques à celles utilisées dans l'exemple 4, à la différence que, cette fois, la charge était constituée d'un mélange de 180 g de poivre et de 20 g de paprika. Après avoir passé 8,4 kg de dioxyde de carbone, on a obtenu 412 g de poudre rouge très homogène en l'absence de grumeau ou agglomérat.

## Revendications

1. Procédé de mise à l'état adsorbé, sur un support poreux, de composés contenus dans un produit, dans lequel, au cours d'une première étape, on réalise l'extraction des composés en mettant en contact avec le produit au moins un fluide solvant à pression supercritique conduisant à l'obtention d'un mélange d'extraits et de fluide solvant, et au cours d'une seconde étape :
- on élimine l'eau contenue dans le mélange d'extraits et de fluide solvant, en faisant percoler ce dernier sur un lit de produit adsorbant apte à fixer cette eau de façon sélective.
- on règle les conditions de température et de pression de façon à obtenir, dans une enceinte (9), deux phases, à savoir une première phase essentiellement constituée du fluide solvant à l'état gazeux et une seconde phase constituée d'un mélange de liquides formé de fluide solvant et des extraits du produit,
- on fait percoler ces deux phases à travers un support poreux apte à adsorber les extraits,
- on vaporise le fluide solvant contenu dans la seconde phase.

2. Procédé selon la revendication 1 **caractérisé en ce que** le fluide solvant est constitué de dioxyde de carbone, de protoxyde d'azote ou d'un hydrocarbure léger ayant de 2 à 8 atomes de carbone.

3. Procédé suivant l'une des revendications précédentes **caractérisé en ce que** le fluide solvant est un fluide pur.

4. Procédé selon l'une des revendications 1 ou 2 **caractérisé en ce que** le fluide solvant est additionné d'au moins un co-solvant.

5. Procédé suivant la revendication 4 **caractérisé en ce que** le co-solvant est constitué d'un alcool et préférablement d'éthanol, et/ou d'une cétone et préférablement d'acétone, et/ou d'un ester et préférablement d'acétate d'éthyle.

6. Procédé selon l'une des revendications précédentes **caractérisé en ce que** la première étape d'extraction est réalisée à une pression comprise entre 7,4 MPa et 80 MPa, et préférablement entre 10 MPa et 40 MPa, et à une température comprise entre 0°C et 80°C.

7. Procédé selon l'une des revendications précédentes **caractérisé en ce que** la percolation des deux phases à travers le support poreux est effectuée à une pression comprise entre 1 MPa et 10 MPa, et préférablement entre 4 MPa et 8 Mpa, et à une température comprise entre 0°C et 80°C.

8. Installation d'extraction/imprégnation comprenant un extracteur (1) et une enceinte d'imprégnation (9) contenant un milieu poreux, l'extracteur (1) contenant un produit dont on souhaite extraire les composés, qui est traversé à cet effet par au moins un fluide solvant à pression supercritique, **caractérisé en ce qu'**il comporte successivement, en aval de l'extracteur (1), des moyens (5) d'élimination de l'eau contenue dans les composés extraits qui sont constitués d'un lit d'adsorbant sélectif, un échangeur réchauffeur (7) des moyens de détente (8) et des moyens d'apport d'enthalpie (11) aptes à créer, dans l'enceinte d'imprégnation (9) deux phases, à savoir une première phase essentiellement constituée du fluide solvant à l'état gazeux et une seconde phase constituée d'un mélange de liquides formé de fluide solvant et des extraits du produit, de façon à réaliser l'adsorption par le milieu poreux des composés extraits.

9. Installation suivant la revendication 8 **caractérisée en ce que** les moyens d'apport d'enthalpie sont constitués d'une double enveloppe (11) à circulation de fluide caloporteur.

10. Installation suivant l'une des revendications 8 ou 9 **caractérisée en ce que** l'extracteur (1) est constitué d'une colonne de fractionnement fonctionnant à contre-courant, adaptée au traitement de matières premières liquides.

11. Installation suivant l'une des revendications 8 à 10 **caractérisée en ce qu'**elle comporte des moyens d'injection d'un co-solvant organique au sein du fluide solvant.

## Patentansprüche

1. Verfahren zur Adsorption auf einem porösen Träger von in einem Produkt enthaltenen Verbindungen, bei welchem bei einem ersten Schritt eine Extraktion von Verbindungen bewerkstelligt wird, **dadurch**, dass das Produkt mit wenigstens einem als Lösungsmittel dienenden Fluid auf überkritischen Druck in Kontakt gebracht wird, wodurch man ein Gemisch erhält, das aus den Extrakten und dem als Lösungsmittel dienenden Fluid besteht, und bei einem zweiten Schritt:
- das Wasser eliminiert wird, das in dem Gemisch aus Extrakten und dem als Lösungsmittel dienenden Fluid enthalten ist, indem man letzteres auf einem Bett perkolieren lässt, das aus einem adsorbierenden Produkt besteht, das geeignet ist, das Wasser selektiv zu binden,
- die Temperatur- und Druckbedingungen derart geregelt werden, dass in einem Behälter (9) zwei Phasen erhalten werden, d.h. eine erste Phase, die im Wesentlichen aus einem als Lösungsmittel dienenden Fluid in gasförmigem Zustand besteht, und eine zweite Phase, die aus einem Gemisch von Flüssigkeiten besteht, das durch das als Lösungsmittel dienende Fluid und die Extrakte des Produktes gebildet ist,
- die zwei Phasen durch einen porösen Träger perkoliert werden, der zur Adsorption der Extrakte in der Lage ist,
- das in der zweiten Phase enthaltene, als Lösungsmittel dienende Fluid verdampft wird.

2. Verfahren nach Anspruch 1, **dadurch gekennzeichnet, dass** das als Lösungsmittel dienende Fluid aus Kohlendioxid, Stickstoffoxid oder einem leichten Kohlenwasserstoff mit 2 bis 8 Kohlenstoffatomen besteht.

3. Verfahren nach einem der vorhergehenden Ansprüche, **dadurch gekennzeichnet, dass** das als Lösungsmittel dienende Fluid ein reines Fluid ist.

4. Verfahren nach einem der Ansprüche 1 oder 2, **dadurch gekennzeichnet, dass** dem als Lösungsmittel dienenden Fluid mindestens ein Cosolvens zugesetzt ist.

5. Verfahren nach Anspruch 4, **dadurch gekennzeichnet, dass** das Cosolvens aus einem Alkohol und bevorzugt Ethanol, und/oder einem Keton und bevorzugt Aceton, und/oder einem Ester und bevorzugt einem Ethylacetat besteht.

6. Verfahren nach einem der vorhergehenden Ansprüche, **dadurch gekennzeichnet, dass** der erste Extraktionsschritt bei einem Druck zwischen 7,4 MPa und 80 MPa, und vorzugsweise zwischen 10 MPa und 40 MPa, und bei einer Temperatur zwischen 0 °C und 80 °C durchgeführt wird.

7. Verfahren nach einem der vorhergehenden Ansprüche, **dadurch gekennzeichnet, dass** das Perkolieren der zwei Phasen durch den porösen Träger bei einem Druck zwischen 1 MPa und 10 MPa, und vorzugsweise zwischen 4 MPa und 8 MPa, und einer Temperatur zwischen 0 °C und 80 °C durchgeführt wird.

8. Anlage zur Extraktion/Imprägnierung, die einen Extraktor (1) und einen Imprägnierungsbehälter (9) aufweist, der ein poröses Medium enthält, wobei der Extraktor (1) ein Produkt enthält, aus dem man die Verbindungen extrahieren möchte, und der zu diesem Zweck von mindestens einem als Lösungsmittel dienenden Fluid auf überkritischem Druck durchströmt wird, **dadurch gekennzeichnet, dass** sie in Strömungsrichtung hinter dem Extraktor
(1) nacheinander aufweist: eine Einrichtung (5) zum Eliminieren des in den extrahierten Verbindungen enthaltenen Wassers, die aus einem selektiven Adsorberbett besteht, einen aufheizenden Wärmetauscher (7), eine Entspannungseinrichtung (8) und eine Einrichtung zur Enthalpiezuführung (11), die in der Lage ist, im Imprägnierungsbehälter (9) zwei Phasen zu erzeugen, und zwar eine erste Phase, die im Wesentlichen aus dem als Lösungsmittel dienenden Fluid in gasförmigem Zustand besteht, und eine zweite Phase, die aus einem Gemisch von Flüssigkeiten besteht, das durch das als Lösungsmittel dienende Fluid und die Extrakte des Produktes gebildet ist, derart, dass eine Adsorption der extrahierten Verbindungen mittels des porösen Mediums erfolgt.

9. Anlage nach Anspruch 8, **dadurch gekennzeichnet, dass** die Enthalpiezuführungseinrichtung aus einer doppelten Umhüllung (11) besteht, in der ein Wärmeaustauschfluid zirkuliert.

10. Anlage nach einem der Ansprüche 8 oder 9, **dadurch gekennzeichnet, dass** der Extraktor (1) aus einer Gegenstrom-Fraktionierkolonne besteht, die zur Behandlung flüssiger Ausgangsmaterialien geeignet ist.

11. Anlage nach einem der Ansprüche 8 bis 10, **dadurch gekennzeichnet, dass** sie eine Einrichtung zum Einbringen eines organischen Cosolvens in das als Lösungsmittel dienende Fluid aufweist.

## Claims

1. Method for setting in adsorbed state, on a porous support, compounds contained in a product, in which, in the course of a first step, the extraction of the compounds is effected by placing in contact with the product at least one solvent fluid at supercritical pressure leading to obtaining a mixture of extracts and of solvent fluid, and, in the course of a second step:
- the water contained in the mixture of extracts and solvent fluid is eliminated by percolating the latter over a bed of adsorbent product adapted to fix this water selectively,
- the temperature and pressure conditions are adjusted so as to obtain, in an enclosure (9), two phases, namely a first phase essentially constituted by the solvent fluid in the gaseous state and a second phase constituted by a mixture of liquids formed of solvent fluid and extracts of the product,
- these two phases are percolated through a porous support adapted to adsorb the extracts,
- the solvent fluid contained in the second phase is vaporized.

2. Method according to Claim 1, **characterized in that** the solvent fluid is constituted by carbon dioxide, nitrogen protoxide or a light hydrocarbon having from 2 to 8 carbon atoms.

3. Method according to one of the preceding Claims, **characterized in that** the solvent fluid is a pure fluid.

4. Method according to one of Claims 1 or 2, **characterized in that** the solvent fluid has at least one co-solvent added thereto.

5. Method according to Claim 4, **characterized in that** the co-solvent is constituted by an alcohol and preferably ethanol, and/or a ketone and preferably acetone, and/or an ester and preferably ethyl acetate.

6. Method according to one of the preceding Claims, **characterized in that** the first step of extraction is carried out at a pressure included between 7.4 MPa and 80 MPa, and preferably between 10 MPa and 40 MPa, and at a temperature included between 0°C and 80°C.

7. Method according to one of the preceding Claims, **characterized in that** the percolation of the two phases through the porous support is effected at a pressure included between 1 MPa and 10 MPa and preferably between 4 MPa and 8 MPa, and at a temperature included between 0°C and 80°C.

8. Installation for extraction/impregnation comprising an extractor (1) and an impregnation enclosure (9) containing a porous medium, the extractor (1) containing a product from which it is desired to extract the components, which is traversed to that end by at least one solvent fluid at supercritical pressure, **characterized in that** it comprises successively, downstream of the extractor (1), means (5) for eliminating the water contained in the extracted compounds which are constituted by a bed of selective adsorbent, an exchanger-heater (7), pressure reduction means (8) and means (11) for supplying enthalpy adapted to create, in the impregnation enclosure (9), two phases, namely a first phase essentially constituted by the solvent fluid in the gaseous state and a second phase constituted by a mixture of liquids formed of solvent fluid and extracts of the product, so as to effect the adsorption by the porous medium of the extracted compounds.

9. Installation according to Claim 8, **characterized in that** the enthalpy supply means are constituted by a double envelope (11) with circulation of heat-transfer fluid.

10. Installation according to one of Claims 8 or 9, **characterized in that** the extractor (1) is constituted by a fractionation column operating in counterflow, adapted for the treatment of liquid raw materials.

11. Installation according to one of Claims 8 to 10, **characterized in that** it comprises means for injecting an organic co-solvent within the solvent fluid.
